# EUROPÄISCHE PATENTSCHRIFT

(11) **EP 0 695 338 B1**
(45) Veröffentlichungstag und Bekanntmachung des Hinweises auf die Patenterteilung: **06.11.1996**
(21) Anmeldenummer: 94913569.3
(22) Anmeldetag: 11.04.1994
(51) Int. Cl.: C10L 1/22, C10M 133/54, C10M 133/58, C10M 133/04, C10M 133/38, C10L 10/00

(54) **POLY-1-N-ALKENAMINE UND DIESE ENTHALTENDE KRAFT- UND SCHMIERSTOFFZUSAMMENSETZUNGEN**
POLY-1-N-ALKENE AMINES AND MOTOR FUEL AND LUBRICANT COMPOSITIONS CONTAINING THEM
POLY-1-N-ALCENAMINES ET COMPOSITIONS DE CARBURANT ET DE LUBRIFIANT LES CONTENANT

(30) Priorität: 22.04.1993 DE 4313088
(43) Veröffentlichungstag der Anmeldung: 07.02.1996
(73) Patentinhaber: BASF AKTIENGESELLSCHAFT, 67056 Ludwigshafen (DE)
(72) Erfinder: MUELLER, Hans-Joachim, D-67269 Gruenstadt (DE); MARCZINKE, Bernd, Lothar, D-67346 Speyer (DE); KLIMESCH, Roger, D-64665 Alsbach-Haehnlein (DE); ROEPER, Michael, D-67157 Wachenheim (DE); FRANZ, Lothar, D-67112 Mutterstadt (DE); SCHREYER, Peter, D-69469 Weinheim (DE); THOMAS, Juergen, D-67136 Fussgoenheim (DE); MOHR, Juergen, D-67269 Gruenstadt (DE); OPPENLAENDER, Knut, D-67061 Ludwigshafen (DE); GUENTHER, Wolfgang, D-67582 Mettenheim (DE)
(86) Internationale Anmeldenummer: EP9401113
(87) Internationale Veröffentlichungsnummer: WO9424231

(56) Entgegenhaltungen:
- EP-A- 0 244 616
- EP-A- 0 268 214
- EP-A- 0 440 508
- US-A- 3 565 804

## Beschreibung

Die Erfindung betrifft Poly-1-n-alkenamine sowie Kraft- und Schmierstoffzusammensetzungen, die Poly-1-n-alkenamine enthalten.

Polybutenylamine und deren Verwendung als Kraftstoff- und Schmierstoffadditive sind schon sehr lange bekannt und beispielsweise in der US-A-3 275 554 und der DE-A-2 125 039 beschrieben.

Die Polybutenylamine des Standes der Technik werden durch Halogenierung von Polybutenen und Umsetzung der Halogenide mit Aminen hergestellt. Bei der Herstellung dieser Produkte entsteht ionogenes Halogen, das möglichst weitgehend entfernt werden muß.

Es hat daher im Stand der Technik nicht an Versuchen gefehlt, die bekannten Produkte zu verbessern, zumal die Beseitigung des ionogenen Halogens nicht nur aufwendige Maßnahmen erfordert, sondern auch in den Umsetzungsprodukten stets beachtliche Halogenmengen verbleiben, vgl. DE-A-2 245 918.

Aus der EP 244 616 B1 sind Polyisobutenamine bekannt, die als Kraft- und Schmierstoffadditive hervorragend geeignet sind. Die zur Herstellung dieser Additive erforderlichen Polyisobutene sind jedoch bereits aufgrund des lediglich durch aufwendige Verfahren verfügbaren Ausgangsprodukts Isobuten nur schwer zugänglich.

Der Erfindung lag daher die Aufgabe zugrunde, Kraft- oder Schmierstoffzusammensetzungen zur Verfügung zu stellen, die die Ablagerungen im Einlaßsystem von Ottomotoren verhindern, eine besonders gute Dispersantwirkung aufweisen und zudem technisch leicht zugänglich sind.

Diese Aufgabe wird gelöst mit einer Kraft- oder Schmierstoffzusammensetzung, die dadurch gekennzeichnet ist, daß sie mindestens ein Poly-1-n-alkenamin der allgemeinen Formel I enthält 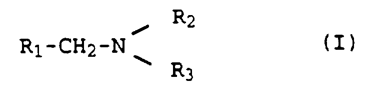 worin
- R₁: einen von einem oder mehreren 1-n-Alkenen mit 3-6 Kohlenstoffatomen und 0-50 Gew.-% Ethen abgeleiteten Poly-1-n-alkenrest bedeutet und
- R₂ und R₃,: die gleich oder verschieden sein können, für Wasserstoff, aliphatische oder aromatische Kohlenwasserstoffe, primäre oder sekundäre, aromatische oder aliphatische Aminoalkylenreste oder Polyaminoalkylenreste, Polyoxyalkylenreste, Heteroaryl- oder Heterocycylreste stehen, oder gemeinsam mit dem Stickstoffatom, an das sie gebunden sind, einen Ring bilden, in dem noch weitere Heteroatome vorhanden sein können.

Nach einer bevorzugten Ausführungsform der Erfindung wird eine Kraft- oder Schmierstoffzusammensetzung enthaltend mindestens ein Poly-1-n-alkenamin der allgemeinen Formel I zur Verfügung gestellt, in der R₁ die oben angegebene Bedeutung hat und worin R₂ und R₃ gleich oder verschieden sind und jeweils Wasserstoff, Alkyl, Aryl, Hydroxyalkyl, einen Aminoalkylenrest der allgemeinen Formel II 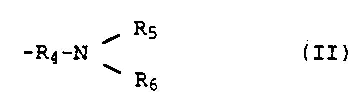 worin
- R₄: für einen Alkylenrest steht und
- R₅ und R₆,: die gleich oder verschieden sind, für Wasserstoff, Alkyl, Aryl oder Hydroxyalkyl stehen oder einen Polyaminoalkylenrest der allgemeinen Formel III 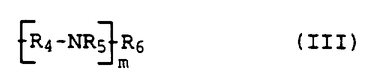 worin die Reste R₄ jeweils gleich oder verschieden sind und die Reste R₅ jeweils gleich oder verschieden sind und die Reste R₄, R₅ und R₆ die zuvor genannten Bedeutungen besitzen, und m für eine ganze Zahl von 1 bis 7 steht, oder einen Polyalkylenrest der allgemeinen Formel IV 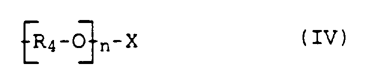 worin die Reste R₄ jeweils gleich oder verschieden sein können und die vorstehende Bedeutung besitzen, X für C₁-C₆-Alkyl oder H steht und n eine ganze Zahl zwischen 1 und 30 darstellt,
bedeuten, oder worin R₂ und R₃ zusammen mit dem Stickstoffatom, an das sie gebunden sind, einen Morpholinylrest, Pyridylrest, Piperidylrest, Pyrrolylrest, Pyrimidinylrest, Pyrrolinylrest, Pyrrolidinylrest, Pyrazinylrest oder Pyridazinylrest darstellen.

Nach einer besonders bevorzugten Ausführungsform wird eine Kraft- oder Schmierstoffzusammensetzung, enthaltend mindestens ein Poly-1-n-alkenamin der allgemeinen Formel I, zur Verfügung gestellt, 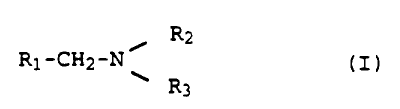 worin
- R₁: einen von einem oder mehreren 1-n-Alkenen mit 3-6 Kohlenstoffatomen und 0-50 Gew.-% Ethen abgeleiteten Poly-1-alkenrest mit 20 bis 400 Kohlenstoffatomen darstellt, und
- R₂ und R₃,: die gleich oder verschieden sind, Wasserstoff, C₁-C₁₀-Alkyl, Phenyl, Naphthyl, C₁-C₁₀-Hydroxyalkyl, einen Aminoalkylenrest der allgemeinen Formel II 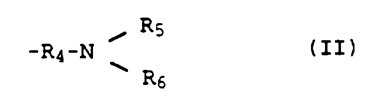 worin R₄ für einen C₂-C₁₀-Alkylenrest steht und

R₅ und R₆, die gleich oder verschieden sind, für Wasserstoff, C₁-C₁₀-Alkyl, Phenyl, Naphthtyl oder C₁-C₁₀-Hydroxyalkyl stehen,
einen Polyaminoalkylenrest der allgemeinen Formel III 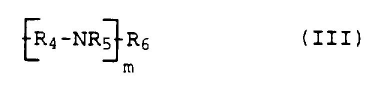 worin die Reste R₄ jeweils gleich oder verschieden sind, die Reste R₅ jeweils gleich oder verschieden sind, die Reste R₄, R₅ und R₆ die vorstehenden Bedeutungen besitzen und m eine ganze Zahl von 1 bis 7 darstellt, oder
einen Polyoxyalkylenrest der allgemeinen Formel IV 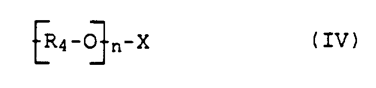 worin die Reste R₄ jeweils gleich oder verschieden sind und die vorstehende Bedeutung besitzen, X für C₁-C₆-Alkyl oder H steht und n eine ganze Zahl zwischen 1 und 30 darstellt,
bedeuten, oder worin R₂ und R₃ zusammen mit dem Stickstoffatom, an das sie gebunden sind, einen Morpholinylrest darstellen.

Nach einer weiteren besonders bevorzugten Ausführungsform wird eine Kraft- oder Schmierstoffzusammensetzung, enthaltend mindestens ein Poly-1-n-alkenamin der allgemeinen Formel I zur Verfügung gestellt, worin der Rest R₁ einen von einem oder mehreren 1-n-Alkenen mit 3-4 Kohlenstoffatomen und 0-40 Gew.-% Ethen abgeleiteten Poly-1-n-alkenrest, insbesondere mit 32 bis 200 Kohlenstoffatomen, darstellt, und R₂ und R₃ gleich oder verschieden sind und Wasserstoff, Methyl, Ethyl, n-Propyl, i-Propyl, n-Butyl, sec.-Butyl, i-Butyl, tert.-Butyl, Pentyl, Hexyl, Phenyl 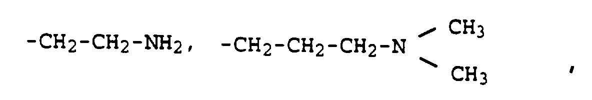 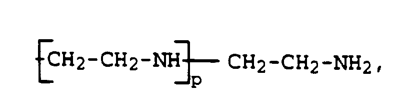 worin p für eine ganze Zahl von 1 bis 7, insbesondere 1 bis 3, steht, 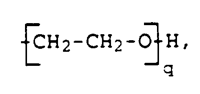 worin q für eine ganze Zahl zwischen 1 und 30 steht, bedeuten, oder gemeinsam mit dem Stickstoffatom, an das sie gebunden sind, einen Morpholinylrest darstellen.

Besonders zweckmäßig sind Kraft- oder Schmierstoffzusammensetzungen, enthaltend mindestens ein Poly-1-n-alkenamin der allgemeinen Formel I, das erhältlich ist durch Polymerisation von 1-n-Alkenen in Gegenwart eines Metallocen-Katalysators der allgemeinen Formel V

CpₘMXₙYᵣ V

in der Cp ein gegebenenfalls substituierter Cyclopentadienring, M ein Übergangsmetall der 4 b-Gruppe, X Wasserstoff oder ein C₁-C₆-Alkylrest und Y ein Halogen bedeuten und worin m=1-3, n=0-3 sowie r=0-3 und m+n+r der Wertigkeit von M entsprechen, anschließender Hydroformylierung des gebildeten Poly-1-n-alkens und hydrierender Aminierung des hydroformylierten Reaktionsprodukts.

Besonders bevorzugt sind Kraft- oder Schmierstoffzusammensetzungen, enthaltend ein Poly-1-n-alkenamin I, worin sich R₁ von einem Polypropen oder einem Ethen-1-Buten-Copolymeren ableitet.

Sofern es sich bei der Erfindung um eine Kraftstoffzusammensetzung, insbesondere um einen Kraftstoff für Verbrennungsmotoren, handelt, kann das Poly-1-n-alkenamin der allgemeinen Formel I beispielsweise in einer Menge von 10-5000 mg/kg, insbesondere 100-800 mg/kg Kraftstoff vorliegen.

In der erfindungsgemäßen Schmierstoffzusammensetzung kann das Poly-1-n-alkenamin beispielsweise in einer Menge von 0,5-5 Gew.-%, insbesondere 1-3 Gew.-%, bezogen auf das Gesamtgewicht der Zusammensetzung, vorliegen.

Die Erfindung betrifft auch Poly-1-n-alkenamine der allgemeinen Formel I 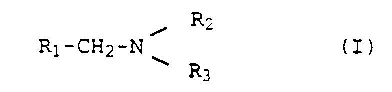 worin R₁ einen von einem oder mehreren 1-n-Alkenen mit 3-6 Kohlenstoffatomen und 0-50 Gew.-% Ethen, insbesondere einen von Propen oder einem Ethen/1-Buten-Gemisch, abgeleiteten Poly-1-n-alkenrest bedeutet und
- R₂ und R₃,: die gleich oder verschieden sind, Wasserstoff, C₁-C₁₀-Alkyl, Phenyl, Naphthyl, C₁-C₁₀-Hydroxyalkyl, einen Aminoalkylenrest der allgemeinen Formel II 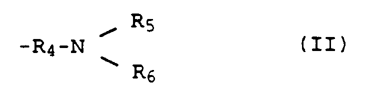
worin
R₄ für einen C₁-C₁₀-Alkylenrest und R₅ und R₆, die gleich oder verschieden sind, für Wasserstoff, C₁-C₁₀-Alkyl, Phenyl, Naphthyl oder C₁-C₁₀-Hydroxyalkyl stehen, einen Polyaminoalkylenrest der allgemeinen Formel III 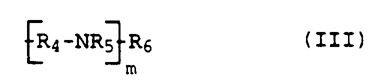 worin die Reste R₄ jeweils gleich oder verschieden sind, die Reste R₅ jeweils gleich oder verschieden sind, die Reste R₄, R₅ und R₆ die vorstehenden Bedeutungen besitzen und m eine ganze Zahl von 1 bis 7 darstellt, oder einen Polyoxyalkylenrest der allgemeinen Formel IV 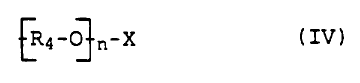 worin die Reste R₄ jeweils gleich oder verschieden sind und die vorstehende Bedeutung besitzen, X für C₁-C₆-Alkyl oder H steht und n eine ganze Zahl zwischen 1 und 30 darstellt, bedeuten, oder worin R₂ und R₃ zusammen mit dem Stickstoffatom, an das sie gebunden sind, einen Ring bilden, in dem noch weitere Heteroatome vorhanden sein können.

Die Erfindung betrifft schließlich noch die Verwendung der Poly-1-n-alkenamine der allgemeinen Formel I, worin R_{1,} R₂ und R₃ die oben angegebenen Bedeutungen haben, als Additive in Kraft-oder Schmierstoffzusammensetzungen, insbesondere für Verbrennungsmotoren.

Die Verbindungen der allgemeinen Formel I lassen sich beispielsweise dadurch herstellen, daß man zunächst 1-n-Alkene in Gegenwart eines Metallocen-Katalysators der allgemeinen Formel V

CpₘMXₙYᵣ V

in der Cp eine unsubstituierte Cyclopentadienyl-Einheit und/oder eine Mono-C₁- bis C₄-alkyl-cyclopentadienyl-Einheit, M ein Zirkonium- oder Hafniumatom ist und in der die Liganden X für Hydridund/oder Halogenionen und /oder eine Methylgruppe stehen, und in Gegenwart eines Aluminoxan-Cokatalysators polymerisiert, dabei den Katalysator bezüglich des Aluminoxan-Cokatalysators in einem Mengenverhältnis einsetzt, das einem M/Al-Atomverhaltnis von 1:250 bis 1:1000 entspricht, und Temperaturen von 50 bis 110°C und einen Druck von 30 bis 100 bar anwendet.

Bei den Katalysatoren V handelt es sich um sogenannte Zirkonocene und Hafnocene, mithin um Komplexe des vierwertigen Zirkoniums und Hafniums, bei denen das Metallatom M sandwichartig zwischen zwei unsubstituierten und/oder C₁-bis C₄-Monoalkyl-substituierte Cyclopentadienyl-Gruppen Cp gebunden ist, wobei die restlichen Valenzen des Zentralatoms M durch Hydrid- und/oder Halogenionen und/ oder durch Methylgruppen abgesättigt sind. Besonders bevorzugt werden solche Zirkonocen- und Hafnocenkatalysatoren im erfindungsgemäßen Verfahren verwendet, deren Cyclopentydienyl-Gruppen unsubstituiert sind. Als Halogenionen können sowohl Fluor-, Chlor-, Brom- und/oder Jodanionen an das Metallatom gebunden sein.

### Beispiele für geeignete Katalysatoren sind:

Cp₂ZrF₂, Cp₂ZrCl₂, Cp₂ZrCl₂, Cp₂ZrJ₂, Cp₂ZrCl, Cp₂Zr(CH₃)Cl, Cp₂Zr(CH₃)₂, Cp₂HfF₂, Cp₂HfCl₂, Cp₂HfBr₂, Cp₂HfJ₂, Cp₂HfHCl, Cp₂Hf(CH₃)Cl, Cp₂Hf(CH₃)₂.

Zweckmäßigerweise wird bei der Oligomerisierung nur ein Katalysator eingesetzt, es ist aber auch möglich, Mischungen verschiedener Katalysatoren zu verwenden. Bevorzugte Liganden X sind Chlorid, Hydrid und die Methylgruppe, als Zentralatom M wird für die Katalysatoren V Zirkonium besonders bevorzugt. Besonders bevorzugt wird Zirkonocenchlorid der Formel Cp₂ZrCl₂ als Katalysator benutzt, dessen Cyclopentadienylgruppen unsubstituiert sind.

Die Katalysatoren können auf einfache Weise nach bekannten Verfahren, z.B. nach Brauer (Hrsg.): Handbuch der Präparativen, Anorganischen Chemie, Band 2, 3.Auflage, Seite 1395 bis 1397, Enke, Stuttgart 1978 synthetisiert werden.

Als Cokatalysatoren werden aluminiumorganische Verbindungen, vorzugsweise Aluminoxane verwendet. Aluminoxane bilden sich bei der partiellen Hydrolyse aluminiumorganischer Verbindungen, beispielsweise solcher der allgemeinen Formeln AlR₃, AlR₂Y und Al₂R₃Y₃, in denen die Reste R z.B. für C₁- bis C₁₀-Alkylgruppen, vorzugsweise C₁- bis C₅-Alkylgruppen, für C₃- bis C₁₀-Cycloalkylgruppen, C₇- bis C₁₂-Aralkyl- oder Alkarylgruppen und/oder eine Phenyl- oder Naphthylgruppe stehen können und in denen Y ein Wasserstoffatom, ein Halogenatom, vorzugsweise ein Chlor- oder Bromatom, oder eine C₁- bis C₁₀-Alkoxygruppe, vorzugsweise eine Methoxy- oder Ethoxygruppe, sein kann. Die partielle Hydrolyse derartiger aluminiumorganischer Verbindungen kann nach verschiedenerlei Verfahren, z.B. nach dem Verfahren der DE-A 3 240 383 oder nach dem in EP-A 268 214 angegebenen, erfolgen. Die dabei entstehenden, sauerstoffhaltigen Aluminoxane sind im allgemeinen keine einheitlichen Verbindungen, sondern Oligomerengemische der allgemeinen Formel VI 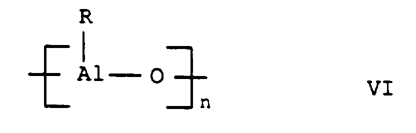 in der in der Regel n eine Zahl von 6 bis 20 ist und R die oben genannte Bedeutung hat. Werden aluminiumorganische Verbindungen mit verschiedenen Resten R oder Gemische aluminiumorganischer Verbindungen mit unterschiedlichen Resten R hydrolysiert, so entstehen Aluminoxane mit verschiedenerlei Resten R, die ebenfalls als Cokatalysator eingesetzt werden können. Zweckmaßigerweise werden allerdings Aluminoxane als Cokatalysatoren benutzt. Als bevorzugtes Aluminoxan dient Methylaluminoxan. Da die als Cokatalysatoren verwendeten Aluminoxane, bedingt durch ihre Herstelgenden die Molarität von Aluminoxanlösungen auf deren Aluminiumgehalt bezogen.

Zur Polymerisierung wird der Katalysator bezüglich des Cokatalysators in einer Menge eingesetzt, die einem M/Al-Atomverhältnis von im allgemeinen 1:250 bis 1:1000, vorzugsweise von 1:300 bis 1:600 und besonders von 1:400 bis 1:500 entspricht.

Die Polymerisierung des 1-n-Alkens wird vorteilhaft in flüssiger Phase und in einem Lösungsmittel, zweckmäßigerweise unter Verwendung geringer Mengen eines Lösungsmittels, vorzugsweise eines aliphatischen oder aromatischen Kohlenwasserstoffes, wie Benzol, Toluol, Xylol, Ethylbenzol, Cumol, Naphthalin, Tetralin, Hexan, Heptan, Octan, Isooctan, Nonan, Decan, Dodecan, Cyclohexan, Decalin, Petrolether oder Ligroin vorgenommen. Als besonders bevorzugte Lösungsmittel werden Toluol und Xylol verwendet. In diesem Verfahren werden Lösungsmittel/l-n-Alken-Volumenverhältnisse von im allgemeinen 1:20 bis 1:500, vorzugsweise von 1:30 bis 1:200 und besonders bevorzugt von 1:40 bis 1:100 eingestellt, wobei sich das Volumen des 1-n-Alkens auf dessen Volumen beim jeweils angewandten Druck bezieht. Unter den angewandten Bedingungen ist das 1-n-Alken flüssig.

Die Polymerisierung wird im allgemeinen bei Temperaturen von 50 bis 110°C, besonders bevorzugt bei 60 bis 90°C und bei einem Druck von 30 bis 100, vorzugsweise von 30 bis 50 bar ausgeführt. Das Metallocen/1-n-Alken-Verhältnis ist in der Regel nicht kritisch für das Verfahren, zweckmäßigerweise werden allerdings Metallocen/l-n-Alken-Molverhältnisse von 1:50 bis 1:250000, vorzugsweise von 1:70 bis 1:200000 und insbesondere von 1:90 bis 1:190000 angewandt.

Das Polymerisations-Verfahren kann sowohl chargenweise, z.B. in Rührautoklaven, oder kontinuierlich, beispielsweise in Rohrreaktoren, durchgeführt werden. Nach der Abtrennung des Katalysators durch Destillation der Produkte oder durch dessen Hydrolyse und anschließende Filtration der ausgefallenen Feststoffe wird das Reaktionsgemisch zweckmäßigerweise destillativ, gewünschtenfalls bei vermindertem Druck, aufgearbeitet.

Das bei diesem Verfahren bevorzugt als Rohstoff verwendete Propen kann aus vielerlei Quellen stammen, z.B. von Crack-Gasen, beispielsweise aus Steamcrackern. Ebenso kann Propen verwendet werden, wie es z.B. bei der Propandehydrierung gebildet wird. Der Einsatz von Propen kann in gereinigter Form erfolgen, es kann allerdings auch in Gemischen mit anderen Kohlenwasserstoffen, die sich unter den Bedingungen der Umsetzung inert verhalten, eingesetzt werden.

Das Polymerisations-Verfahren ermöglicht die selektive Herstellung von Poly-1-n-alkenen mit endständigen Doppelbindungen, insbesondere die selektive Herstellung von Propenpolymeren mit hohen Produktivitäten.

Die Poly-1-n-alkene, insbesondere die Copolymere aus Ethen und 1-n-Alkenen, lassen sich auch nach anderen bekannten Verfahren herstellen, wie sie z.B. in der EP 0441 548 Al beschrieben sind. Auch hier wird ein Metallocen-Katalysator in Kombination mit einem Aluminoxan eingesetzt. Als Metallocene werden hierbei ebenfalls Cyclopentadienyl- Übergangsmetall-Verbindungen der Formel V verwendet, wobei als Übergangsmetalle Ti, Zr und Hf bevorzugt sind.

Die so hergestellten Poly-1-n-alkene werden anschließend, gegebenenfalls nach vorheriger Destillation, in an sich bekannter Weise hydroformyliert. Dabei werden die Poly-1-n-alkene mit einem Rhodium- oder Kobaltkatalysator in Gegenwart von CO und H₂ bei Temperaturen zwischen 80 und 200°C und CO/H₂-Drucken von bis zu 600 bar hydroformyliert.

Anschließend wird das Reaktionsprodukt (Oxoprodukt) hydrierend aminiert. Die Aminierungsreaktion wird zweckmäßigerweise bei Temperaturen von 80-200°C und Drucken bis 600 bar, vorzugsweise 80-300 bar, durchgeführt.

Bei der Oxierungs- und Aminierungsreaktion wird zweckmäßigerweise ein geeignetes, inertes Lösungsmittel verwendet, um die Viskosität des Reaktionsgemisches herabzusetzen. Als Lösungsmittel sind vor allem schwefelarme aliphatische, cycloaliphatische und aromatische Kohlenwasserstoffe geeignet. Besonders bevorzugt sind aliphatische Lösungsmittel, die frei von Schwefelverbindungen sind und weniger als 1% Aromaten enthalten. Sie haben den Vorteil, daß bei hohen Aminierungstemperaturen keine Hydrierwärme frei und kein Wasserstoff verbraucht wird. Bei der Aminierungs- und Hydroformylierungsreaktion liegt der Lösungsmittelgehalt je nach Viskosität des Polymeren und des Lösungsmittels zwischen 0 und 70 Gew.-%. Höhere Verdünnungen sind ebenso unwirtschaftlich wie ein Lösungsmittelaustausch zwischen Oxierung und Aminierung.

Das bei der Hydroformylierung entstehende Oxoprodukt liegt normalerweise als Aldehyd/Alkoholgemisch vor. Es kann als Gemisch weiterverarbeitet oder aus Gründen der Lagerstabilität vorher vollständig hydriert werden. Vollständig hydrierte Produkte sind weniger reaktiv.

Die in den erfindungsgemäßen Kraft- oder Schmierstoffen eingesetzten Poly-1-n-alkenamine enthalten aufgrund ihrer Herstellung kein Halogen und weisen zudem keine ungesättigten Anteile auf, was sie für die Verwendung in Kraft- oder Schmierstoffen besonders geeignet macht.

Die erfindungsgemäßen Poly-1-n-alkenamine weisen aufgrund ihrer Struktur sowohl dispergierende als auch Detergenz-Wirkung auf. Das heißt, sie führen als Detergentien zur Reinhaltung von Ventilen und Vergasern bzw. Einspritzsystemen. Als Dispersant tragen sie, über den Brennraum in den Schmiermittelkreislauf des Motors gelangt, zu einer Verbesserung der Schlammdispergierung in Motoröl bei.

Sollen in erster Linie die dispergierenden Eigenschaften der Poly-1-n-alkenamine genutzt werden, so kann man sie auch mit herkömmlichen Detergentien als zusätzlichen Additiven kombinieren.

Als Detergens-Komponente in der Mischung mit den erfindungsgemäßen Stoffen als Dispergatoren kann prinzipiell jedes bekannte der hierfür geeigneten Produkte eingesetzt werden, wie sie z.B. bei J. Falbe, U. Hasserodt, Katalysatoren, Tenside und Mineralöladditive, G. Thieme Verlag Stuttgart 1978, S. 221 f. oder bei K. Owen, Gasoline and Diesel Fuel Additives, John Wiley & Sons 1989, S. 23 ff., beschrieben sind.

Vorzugsweise verwendet man N-haltige Detergentien, z.B. Verbindungen, die eine Amin- oder Amid-Gruppe enthalten. Insbesondere geeignet sind Polyisobutylamine gemäß EP 0 244 616, Ethylendiamintetraessigsäureamide und/oder -imide gemäß EP 0 188 786 oder Polyetheramine gemäß EP 0 244 725, wobei auf die Definitionen in diesen Literaturstellen Bezug genommen wird. Die dort beschriebenen Produkte verfügen herstellungsbedingt ebenfalls über den Vorteil, chlor- bzw. cloridfrei zu sein.

Soll in erster Linie die Detergens-Wirkung der erfindungsgemaßen Verbindungen genutzt werden, so können diese Stoffe auch mit Tragerölen kombiniert werden. Derartige Trägeröle sind bekannt, insbesondere eignen sich Trägeröle auf Polyglykolbasis, z.B. entsprechende Ether und/oder Ester, wie sie in der US 5 004 478 oder der DE 38 38 918 A1 beschrieben sind. Auch Polyoxyalkylenmonoole mit Kohlenwasserstoffendgruppen (US 4 877 416) oder Trägeröle, wie sie in der DE 41 42 241 Al offenbart sind, können eingesetzt werden.

Als Kraftstoffe für Ottomotoren kommen verbleites und insbesondere unverbleites Normal- und Superbenzin in Betracht. Die Benzine können auch andere Komponenten als Kohlenwasserstoffe, z.B. Alkohole wie Methanol, Ethanol, tert.-Butanol sowie Ether, z.B. Methyl-tert.-butylether enthalten. Neben den erfindungsgemäßen zu verwendenden Poly-1-n-alkenaminen enthalten die Kraftstoffe in der Regel noch weitere Zusätze wie Korrosionsinhibitoren, Stabilisatoren, Antioxidantien und/oder weitere Detergentien.

Korrosionsinhibitoren sind meist Ammoniumsalze org. Carbonsäuren, die durch entsprechende Struktur der Ausgangsverbindungen zur Filmbildung neigen. Auch Amine zur Absenkung des pH-Wertes finden sich häufig in Korrosionsinhibitoren. Als Buntmetallkorrosionsschutz werden meist heterocyclische Aromaten eingesetzt.

Die Prüfung der erfindungsgemäßen Poly-1-n-alkenamine bezüglich ihrer Eignung als Ventilreiniger erfolgt mittels Motorentests mit einem 1,2 1 Opel-Kadett-Motor.

### Beispiele

### 1. Herstellung von Poly-1-n-alkenen

1.1 In einem 2 1-Rührautoklaven wurden 30 ml 1,5 molare Methylaluminoxanlösung in Toluol vorgelegt, 900 ml (13,3 Mol) flüssiges Propen aufkondensiert und auf 60°C erwärmt. Dabei stellte sich ein Druck von 20 bar ein. Anschließend wurden 40.5 mg (0,17 mmol) Zirkonocen (Dicyclopentadienyl-zirkoniumdichlorid), gelöst in 7 ml einer 1,5 molaren Methylaluminoxanlösung in Toluol, zugegeben und während einer Zeitdauer von 60 Minuten oligomerisiert. Das Aluminium/Zirkonium-Atomverhältnis betrug 250:1. Es wurde eine Ausbeute von 590 ml Propenoligomere erhalten. Die Produktivität des Katalysators unter den angewandten Reaktionsbedingungen, ausgedrückt als ml Produkt/g Katalysator x h, betrug 11900. Die gaschromatografische Analyse des Produkts ergab die folgende Zusammensetzung:

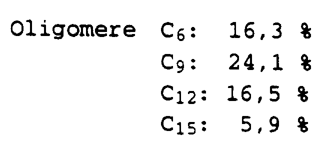

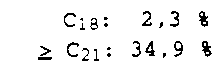

Die infrarot- und NMR-spektroskopische Analyse der erhaltenen Produkte belegt, daß ausschließlich Kohlenwasserstoffe mit endständigen Doppelbindungen, die überwiegend in Vinylidengruppen lokalisiert sind, gebildet wurden.

Entsprechend Beispiel 1 wurden die Beispiele 2 bis 4 durchgeführt, wobei die in der Tabelle angegebenen unterschiedlichen Aluminium/Zirkonium-Atomverhältnisse angewandt wurden.

| Beispiel | Zirkonocen[mmol] | Methylaluminoxan [mmol] | Al/Zr-Atomverhältnis | Produktivität [ml Produkt/g Kat x h] |
|---|---|---|---|---|
| 1.2 | 0,17 | 90 | 530 | 14600 |
| 1.3 | 0,07 | 30 | 430 | 18600 |
| 1.4 | 0,07 | 60,5 | 860 | 20100 |

### Beispiel 1.5

In einem 1 l-Rührautoklaven wurden 30 ml einer 1,5 molaren Methylalumoxanlösung in Toluol vorgelegt, 500 ml (6,3 mol) flüssiges 1-n-Buten aufkondensiert und auf 80°C erwärmt. Dabei stellte sich ein Druck von 13 bar ein. Anschießend wurden 0,1 mol Ethylen zudosiert. Nach Zugabe von 28 mg (0,096 mmol) Zirkonocen wurde 30 Minuten oligomerisiert.

Es wurden 551 ml Buten-Ethylen-Oligomerisat isoliert.

### 2. Hydroformylierung

2.1 Das gemäß Beispiel 1.1 hergestellte Propen-Oligomerisat wurde destilliert und die Fraktion ≥ C₂₁ wurde ohne Lösungsmittel bei zwei verschiedenen Temperaturen (120 und 160°C) bei 280 bar oxiert.
Ausgangsprodukt

| | |
|---|---|
| Iod-Zahl: | 49 g Iod/100 g |

A) Hochdruck-Oxierung bei 120°C
Eine Lösung von 4,5 g 85 %igem Cobaltcarbonyl in 700 g Polypropen [0.18 % Cobalt] wurde in einem 2,5 1 Hubrührautoklaven 5 Stunden bei 120 °C/280 bar CO/H₂ zur Reaktion gebracht. Zur Abtrennung des Cobaltkatalysators wurde das Reaktionsgemisch nach Entspannen mit dem gleichen Volumen einer 10 %igen Essigsäurelösung bei 90 bis 95°C unter Durchleiten von Luft eine Stunde gerührt und die cobalthaltige wäßrige Phase abgetrennt.
Das Oxo-Produkt wies folgende Kennzahlen auf:

| | |
|---|---|
| Iod-Zahl: | 8,6 g Iod/100 g |
| CO-Zahl: | 67 mg KOH/g |
| OH-Zahl : | 1 mg KOH/g |
| | |
| Umsatz : | 80 % |
| Ausbeute : | 85,5 % (säulenchromatographisch bestimmt) |

B) Hochdruck-Oxierung bei 160°C
Versuchsausführung wie A
Kennzahlen von Oxo-Prod.:

| | |
|---|---|
| Iod-Zahl | 0,1 |
| | |
| CO-Zahl | 2,5 sauer heiß |
| CO-Zahl | 2,5 kalt alkalisch |
| OK-Zahl | 40 |
| | |
| Umsatz | 99,8 % |

2.2 Das gemäß Beispiel 1.5 hergestellte Buten-Ethylen-Oligomerisat wurde diskontinuierlich mit Cobalt unter folgenden Bedingungen hydroformyliert:
Das Ausgangsprodukt [Iod-Zahl 53] wurde als 30 %ige Lösung in Toluol eingesetzt.

| | |
|---|---|
| App.: | 2,5 l Hubrüchrautoklav |
| Temp.: | 160°C |
| Druck: | 260-280 bar CO/H₂ 1:1 |
| Cobalt: | 0,18 % im Feed |
| Cobalt als: | Carbonyl |
| Zeit: | 5 Stunden |

Zur Abtrennung des Cobaltkatalysators wurde das Reaktionsgemisch nach Entspannen mit dem gleichen Volumen einer 10 %igen Essigsäurelösung bei 90 bis 95°C unter Durchleiten von Luft eine Stunde gerührt und die cobalthaltige Phase abgetrennt. Anschließend wurde das Lösungsmittel im Vakuum entfernt.

| % a) Ausbeute | % Umsatz | Iod-Zahl | CO-Zahl | OH-Zahl | Säure-Zahl |
|---|---|---|---|---|---|
| 96 | 98,7 | 0,7 | 15 | 55 | 1,2 |

| | | | | | |
|---|---|---|---|---|---|
| a) säulenchromatographisch bestimmt | | | | | |

### 3. Aminierung

3.1 400 g des Hydroformylierungsproduktes nach Beispiel 2.1A wurden im Rührautoklaven mit 760 ml NH3 (flüssig) und 75 g Raney-Nickel versetzt und bei 280 bar H2-Druck 4 h auf 180°C erhitzt. Nach Filtration wies das Produkt folgende Kennzahlen auf:

| | |
|---|---|
| Aminzahl: | 60,5 |
| sek. und tert. Aminzahl: | 1,6 |
| OH-Zahl: | 7,7 |

3.2 300 g des Hydroformylierungsproduktes nach Beispiel 2.1A wurden im Rührautoklaven mit 40 g Diethylentriamin, 150 g Cyclohexan und 50 g Raney-Nickel bei 280 bar H2-Druck 4 h auf 180°C erhitzt. Nach Filtration und Verdampfen des Lösungsmittels wies der Rückstand folgende Kennzahlen auf:

| | |
|---|---|
| Aminzahl: | 115,0 |
| sek. und tert. Aminzahl: | 44,6 |
| OH-Zahl: | 6,1 |

4. Motorversuche
Die Motorversuche wurden in einem Opel-Kadett 1,2 l Motor durchgeführt. Eingesetzter Kraftstoff: Euro-Super bleifrei.

| Additiv | Dosierung | Einlaßventilablagerung in mg | | | | |
|---|---|---|---|---|---|---|
| | | Ventile | 1 | 2 | 3 | 4 |
| Polypropylenamin nach Beispiel 3.1 | 800 ppm | | 0 | 0 | 0 | 0 |
| Grundwert ohne Additiv | | | 830 | 384 | 338 | 750 |

Es zeigt sich somit, daß die erfindungsgemäßen Polyalkenamine eine ausgezeichnete Wirkung als Detergens aufweisen.
5. Tüpfeltest
Es wurde eine 3 gew.-%ige Mischung des Polypropylenamines nach Beispiel 3.1 mit einer Rußdispersion in einem Mineralöl durch Erhitzen auf 50°C für 1 h hergestellt. Die so erhaltene Dispersion wurde auf einem Filterpapier wie ein Chromatogramm entwickelt. Verglichen wird die Fläche von reinem Öl und verteiltem Ruß (Testbeschreibung: "Les Huiles pour Moteurs et le Fraissage des Moteurs", A. Schilling, Vol. 1, S. 89f, 1962)

| Additivgehalt der Rußdispersion | Flächenanteile Ruß |
|---|---|
| [Gew.-%] | [%] |
| 0 | 22 |
| 3 | 45 |

Der Versuch zeigt deutlich die dispergierenden Eigenschaften der erfindungsgemäßen Poly-1-n-alkenamine.

## Patentansprüche

1. Kraft- oder Schmierstoffzusammensetzung, dadurch gekennzeichnet, daß sie mindestens ein Poly-1-n-alkenamin der allgemeinen Formel I enthält 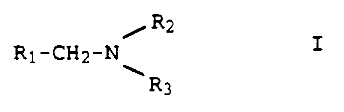 worin
R₁ einen von einem oder mehreren 1-n-Alkenen mit 3-6 Kohlenstoffatomen und 0-50 Gew.-% Ethen abgeleiteten Poly-1-n-alkenrest bedeutet und
R₂ und R₃, die gleich oder verschieden seih können, für Wasserstoff, aliphatische oder aromatische Kohlenwasserstoffreste, primäre oder sekundäre, aromatische oder aliphatische Aminoalkylenreste oder Polyaminoalkylenreste, Polyoxyalkylenreste, Heteroaryl- oder Heterocyclylreste stehen können, oder gemeinsam mit dem Stickstoffatom, an das sie gebunden sind, einen Ring bilden, in dem noch weitere Heteroatome vorhanden sein können.

2. Kraft- oder Schmierstoffzusammensetzung nach Anspruch 1, enthaltend mindestens ein Poly-1-n-alkenamin der allgemeinen Formel I, worin R₁ die in Anspruch 1 angegebene Bedeutung besitzt und
R₂ und R₃ gleich oder verschieden sind und jeweils
Wasserstoff, Alkyl, Aryl, Hydroxyalkyl,
einen Aminoalkylenrest der allgemeinen Formel II 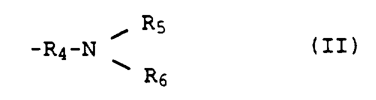 worin R₄ für einen Alkylenrest steht und
R₅ und R₆, die gleich oder verschieden sind, für Wasserstoff, Alkyl, Aryl oder Hydroxyalkyl,
einen Polyaminoalkylenrest der allgemeinen Formel III 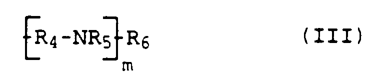 worin die Reste R₄ jeweils gleich oder verschieden sind und die Reste R₅ jeweils gleich oder verschieden sind und die Reste R₄, R₅ und R₆ die zuvor genannten Bedeutungen besitzen, und m für eine ganze Zahl von 1 bis 7 steht,
oder einen Polyoxyalkylenrest der allgemeinen Formel IV 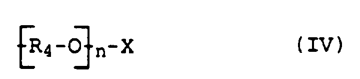 worin die Reste R₄ jeweils gleich oder verschieden sein können und die vorstehende Bedeutung besitzen, X für C₁-C₆-Alkyl oder H steht und n eine ganze Zahl zwischen 1 und 30 darstellt,
bedeuten,
oder worin R₂ und R₃ zusammen mit dem Stickstoffatom, an das sie gebunden sind, einen Morpholinylrest, Pyridylrest, Piperidylrest, Pyrrolylrest, Pyrimidinylrest, Pyrrolinylrest, Pyrrolidinylrest, Pyrazinylrest oder Pyridazinylrest darstellen.

3. Kraft- oder Schmierstoffzusammensetzung nach Anspruch 1, enthaltend mindestens ein Poly-1-n-alkenamin der allgemeinen Formel I 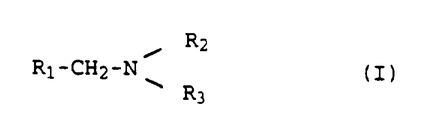 worin
R₁ einen von einem oder mehreren 1-n-Alkenen mit 3-6 Kohlenstoffatomen und 0-50 Gew.-% Ethen abgeleiteten Poly-1-n-alkenrest mit 20 bis 400 Kohlenstoffatomen darstellt, und
R₂ und R₃, die gleich oder verschieden sind, Wasserstoff, C₁-C₁₀-Alkyl, Phenyl, Naphthyl, C₁-C₁₀-Hydroxyalkyl,
einen Aminoalkylenrest der allgemeinen Formel II 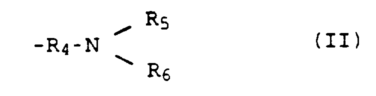 worin
R₄ für einen C₂-C₁₀-Alkylenrest steht und
R₅ und R₆, die gleich oder verschieden sind, für Wasserstoff, C₁-C₁₀-Alkyl, Phenyl, Naphthyl oder C₁-C₁₀-Hydroxyalkyl stehen,
einen Polyaminoalkylenrest der allgemeinen Formel III 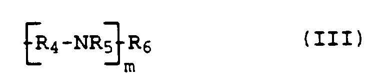
worin
die Reste R₄ jeweils gleich oder verschieden sind,
die Reste R₅ jeweils gleich oder verschieden sind,
die Reste R₄, R₅ und R₆ die vorstehenden Bedeutungen
besitzen und m eine ganze Zahl von 1 bis 7 darstellt, oder
einen Polyoxyalkylenrest der allgemeinen Formel IV 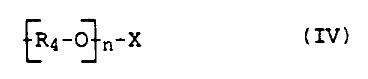 worin die Reste R₄ jeweils gleich oder verschieden sind und die vorstehende Bedeutung besitzen, X für C₁-C₆-Alkyl oder H steht und n eine ganze Zahl zwischen 1 und 30 darstellt,
bedeuten, oder worin R₂ und R₃ zusammen mit dem Stickstoffatom, an das sie gebunden sind, einen Morpholinylrest darstellen.

4. Kraft- oder Schmierstoffzusammensetzung nach Anspruch 3, enthaltend mindestens ein Poly-1-n-alkenamin der allgemeinen Formel I, worin der Rest R₁ einen von einem oder mehreren 1-n-Alkenen mit 3 bis 4 Kohlenstoffatomen und 0 bis 40 Gew.-% Ethen abgeleiteten Poly-1-n-alkenrest darstellt, und
R₂ und R₃ gleich oder verschieden sind und Wasserstoff, Methyl, Ethyl, n-Propyl, i-Propyl, n-Butyl, sec.-Butyl, i-Butyl, tert.-Butyl, Pentyl, Hexyl, Phenyl, 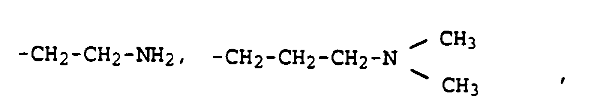 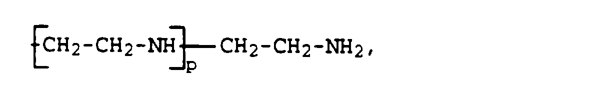 worin p für eine ganze Zahl von 1 bis 7, insbesondere 1 bis 3, steht, 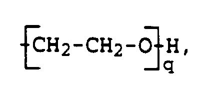 worin q für eine ganze Zahl zwischen 1 und 30 steht, bedeuten, oder gemeinsam mit dem Stickstoffatom, an das sie gebunden sind, einen Morpholinylrest darstellen.

5. Kraft- oder Schmierstoffzusammensetzung nach Anspruch 1, enthaltend mindestens ein Poly-1-n-alkenamin der allgemeinen Formel I, worin der Rest R₁ einen von Propen oder einen von einem Ethen/1-n-Buten-Gemisch abgeleiteten Poly-1-n-alkenrest darstellt.

6. Kraft- oder Schmierstoffzusammensetzung nach Anspruch 1, dadurch gekennzeichnet, daß das Poly-1-n-alkenamin erhältlich ist durch
a) Polymerisation von 1-n-Alkenen in Gegenwart eines Metallocen-Katalysators der allgemeinen Formel V
CpₘMXₙYᵣ V
in der Cp ein gegebenenfalls substituierter Cyclopentadienring, M ein Übergangsmetall der 4 b-Gruppe, X Wasserstoff oder ein C₁-C₆-Alkylrest und Y ein Halogen bedeuten und worin m=1-3, n=0-3 sowie r=0-3 und m+n+r der Wertigkeit von M entsprechen,
b) anschließende Hydroformylierung des Poly-1-n-alkens und
c) hydrierende Aminierung des Reaktionsprodukts aus dem Reaktionsschritt b).

7. Kraftstoff zusammensetzung nach Anspruch 1, dadurch gekennzeichnet, daß sie das Poly-1-n-alkenamin in einer Menge von 10 bis 5000 ppm, insbesondere 100 bis 800 ppm, bezogen auf das Gesamtgewicht der Zusammensetzung, enthält.

8. Schmierstoffzusammensetzung nach Anspruch 1, dadurch gekennzeichnet, daß sie das Poly-1-n-alkenamin in einer Menge von 0,5 bis 5 Gew.-%, insbesondere 1 bis 3 Gew.-%, bezogen auf das Gesamtgewicht der Zusammensetzung, enthält.

9. Poly-1-n-alkenamine der allgemeinen Formel I 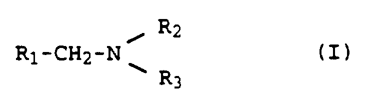 worin R₁ einen von einem oder mehreren 1-n-Alkenen mit 3-6 Kohlenstoffatomen und 0-50 Gew.-% Ethen abgeleiteten Poly-1-n-alkenrest bedeutet und
R₂ und R₃, die gleich oder verschieden sind,
Wasserstoff, C₁-C₁₀-Alkyl, Phenyl, Naphthyl, C₁-C₁₀-Hydroxyalkyl,
einen Aminoalkylenrest der allgemeinen Formel II 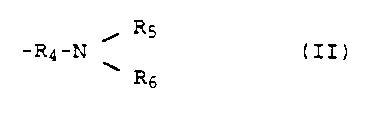 worin
R₄ für einen C₁-C₁₀-Alkylenrest und R₅ und R₆, die gleich oder verschieden sind, für Wasserstoff, C₁-C₁₀-Alkyl, Phenyl, Naphthyl oder C₁-C₁₀-Hydroxyalkyl stehen,
einen Polyaminoalkylenrest der allgemeinen Formel III 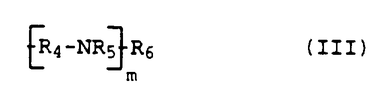 worin die Reste R₄ jeweils gleich oder verschieden sind, die Reste R₅ jeweils gleich oder verschieden sind, die Reste R₄, R₅ und R₆ die vorstehenden Bedeutungen besitzen und m eine ganze Zahl von 1 bis 7 darstellt, oder
einen Polyoxyalkylenrest der allgemeinen Formel IV 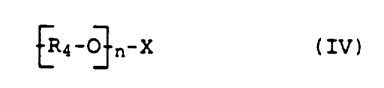 worin die Reste R₄ jeweils gleich oder verschieden sind und die vorstehende Bedeutung besitzen, X für C₁-C₆-Alkyl oder H steht und n eine ganze Zahl zwischen 1 und 30 darstellt,
bedeuten, oder worin R₂ und R₃ zusammen mit dem Stickstoffatom, an das sie gebunden sind, einen Ring bilden, in dem noch weitere Heteroatome vorhanden sein können.

10. Verwendung der Poly-1-n-alkenamine der allgemeinen Formel I gemäß Anspruch 9 als Additive in Kraft- oder Schmierstoffzusammensetzungen, insbesondere für Verbrennungsmotoren.

## Claims

1. A fuel or lubricant composition, which contains at least one poly-1-n-alkenylamine of the formula I 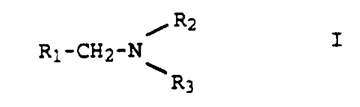 where
R₁ is a poly-1-n-alkene radical derived from one or more 1-n-alkenes of 3-6 carbon atoms and 0-50% by weight of ethene and
R₂ and R₃ may be identical or different and are each hydrogen, aliphatic or aromatic hydrocarbons, primary or secondary, aromatic or aliphatic aminoalkylene radicals or polyaminoalkylene radicals, polyoxyalkylene radicals, heteroaryl or heterocycyl radicals or, together with the nitrogen atom to which they are bonded, form a ring in which further hetero atoms may be present.

2. A fuel or lubricant composition as claimed in claim 1, containing at least one poly-1-n-alkenylamine of the formula I, where R₁ has the meanings stated in claim 1 and
R₂ and R₃ are identical or different and are each hydrogen, alkyl, aryl, hydroxyalkyl, an aminoalkylene radical of the formula II 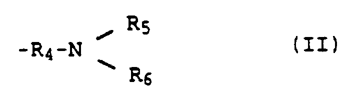 where
R₄ is an alkylene radical and
R₅ and R₆ are identical or different and are each hydrogen, alkyl, aryl or hydroxyalkyl,
a polyaminoalkylene radical of the formula III 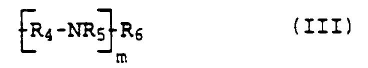 where the radicals R₄ are identical or different, the radicals R₅ are identical or different, R₄, R₅ and R₆ have the abovementioned meanings and m is an integer of from 1 to 7,
or a polyoxyalkylene radical of the formula IV 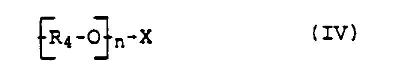 where the radicals R₄ may be identical or different and have the abovementioned meanings, X is C₁-C₆-alkyl or H and n is an integer from 1 to 30,
or where R₂ and R₃, together with the nitrogen atom to which they are bonded, form a morpholinyl, pyridyl, piperidyl, pyrrolyl, pyrimidinyl, pyrrolinyl, pyrrolidinyl, pyrazinyl or pyridazinyl radical.

3. A fuel of lubricant composition as claimed in claim 1, containing at least one poly-1-n-alkenylamine of the formula I 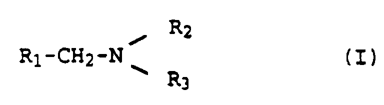 where
R₁ is a poly-1-n-alkene radical of 20 to 400 carbon atoms which is derived from one or more 1-n-alkenes of 3-6 carbon atoms and 0-50% by weight of ethene and
R₂ and R₃ are identical or different and are each hydrogen, C₁-C₁₀-alkyl, phenyl, naphthyl, C₁-C₁₀-hydroxyalkyl, an aminoalkylene radical of the formula II 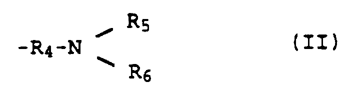 where
R₄ is a C₂-C₁₀-alkylene radical and
R₅ and R₆ are identical or different and are each hydrogen, C₁-C₁₀-alkyl, phenyl, naphthyl or C₁-C₁₀-hydroxyalkyl,
a polyaminoalkylene radical of the formula III 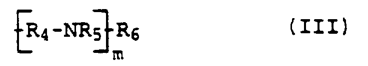 where
the radicals R₄ are identical or different, the radicals R₅ are identical or different, R₄, R₅ and R₆ have the abovementioned meanings and m is an integer of from 1 to 7, or
a polyoxyalkylene radical of the formula IV 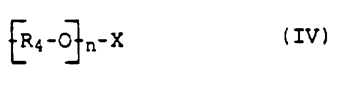 where the radicals R₄ are identical or different and have the abovementioned meanings, X is C₁-C₆-alkyl or H and n is an integer from 1 to 30,
or where R₂ and R₃, together with the nitrogen atom to which they are bonded, form a morpholinyl radical.

4. A fuel or lubricant composition as claimed in claim 3, containing at least one poly-1-n-alkenylamine of the formula I, where R₁ is a poly-1-n-alkene radical derived from one or more 1-n-alkenes of 3 or 4 carbon atoms and from 0 to 40% by weight of ethene, and R₂ and R₃ are identical or different and are each hydrogen, methyl, ethyl, n-propyl, isopropyl, n-butyl, sec-butyl, isobutyl, tert-butyl, pentyl, hexyl, phenyl, 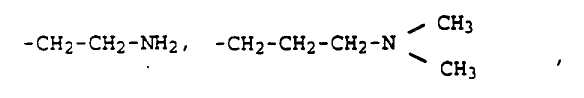 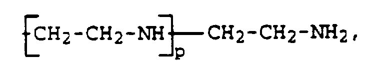 where p is an integer from 1 to 7, in particular from 1 to 3, 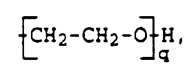 where q is an integer from 1 to 30, or, together with the nitrogen atom to which they are bonded, form a morpholinyl radical.

5. A fuel or lubricant composition as claimed in claim 1, containing at least one poly-1-n-alkenylamine of the formula I, where R₁ is a poly-1-n-alkene radical derived from propene or from an ethene/1-n-butene mixture.

6. A fuel or lubricant composition as claimed in claim 1, wherein the poly-1-n-alkenylamine is obtainable by
a) polymerization of a 1-n-alkene in the presence of a metallocene catalyst of the formula V
CpₘMXₙYᵣ V
where Cp is an unsubstituted or substituted cyclopentadiene ring, M is a transition metal of group 4 b, X is hydrogen or C₁-C₆-alkyl, Y is halogen, m is 1-3, n is 0-3, r is 0-3 and m+n+r correspond to the valency of M,
b) subsequent hydroformylation of the poly-1-n-alkene and
c) amination of the reaction product from reaction step b) under hydrogenating conditions.

7. A fuel composition as claimed in claim 1, which contains the poly-1-n-alkenylamine in an amount of from 10 to 5,000 ppm, in particular from 100 to 800 ppm, based on the total weight of the composition.

8. A lubricant composition as claimed in claim 1, which contains the poly-1-n-alkenylamine in an amount of from 0.5 to 5, in particular from 1 to 3, % by weight, based on the total weight of the composition.

9. A poly-1-n-alkenylamine of the formula I 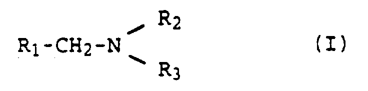 where R₁ is a poly-1-n-alkene radical derived from one or more 1-n-alkenes of 3-6 carbon atoms and 0-50% by weight of ethene and
R₂ and R₃ are identical or different and are each hydrogen, C₁-C₁₀-alkyl, phenyl, naphthyl, C₁-C₁₀-hydroxyalkyl, an aminoalkylene radical of the formula II 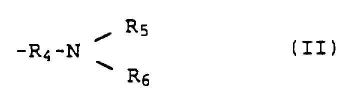 where
R₄ is a C₁-C₁₀-alkylene radical and R₅ and R₆ are identical or different and are each hydrogen, C₁-C₁₀-alkyl, phenyl, naphthyl or C₁-C₁₀-hydroxyalkyl, a polyaminoalkylene radical of the formula III 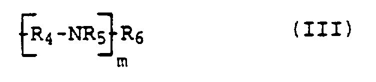 where the radicals R₄ are identical or different, the radicals R₅ are identical or different, R₄, R₅ and R₆ have the above meanings and m is an integer of from 1 to 7, or a polyoxyalkylene radical of the formula IV 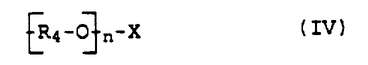 where the radicals R₄ are identical or different and have the above meanings, X is C₁-C₆-alkyl or H and n is an integer of from 1 to 30,
or where R₂ and R₃, together with the nitrogen atom to which they are bonded, form a ring in which further hetero atoms may be present.

10. Use of a poly-1-n-alkenylamine of the formula I as claimed in claim 9 as an additive in fuel or lubricant compositions, in particular for internal combustion engines.

## Revendications

1. Composition de carburant ou de lubrifiant, caractérisée en ce qu'elle contient au moins une poly-1-n-alcénamine de la formule générale I : 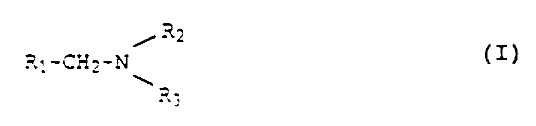 dans laquelle
R₁ représente un reste de poly-1-n-alcène dérivé d'un ou plusieurs 1-n-alcènes comportant de 3 à 6 atomes de carbone, et de 0 à 50% en poids d'éthène, et
R₂ et R₃, qui peuvent être identiques ou différents, représentent des radicaux hydrocarbonés aliphatiques ou aromatiques, des radicaux aminoalkylène primaires ou secondaires, aromatiques ou aliphatiques, ou des radicaux polyaminoalkylène, des radicaux polyoxyalkylène, des radicaux hétéroaryle ou hétérocyclyle, ou forment ensemble avec l'atome d'azote, auquel ils sont attachés, un cycle dans lequel encore d'autre hétéroatomes peuvent être présents.

2. Composition de carburant ou de lubrifiant suivant la revendication 1, contenant au moins une poly-1-n-alcénamine de la formule générale I, dans laquelle R₁ possède les significations qui lui ont été attribuées dans la revendication 1, et
R₂ et R₃ sont identiques ou différents et représentent chacun
un atome d'hydrogène, un radical alkyle, aryle, hydroxyalkyle,
un radical aminoalkylène de la formule générale II : 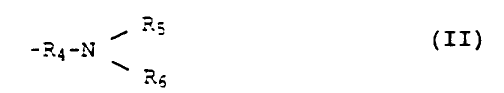
dans laquelle R₄ représente un radical alkylène, et
R₅ et R₆, qui peuvent être identiques ou différents, représentent des atomes d'hydrogène, des radicaux alkyle, aryle, ou hydroxyalkyle,
un radical polyaminoalkylène de la forme générale III : 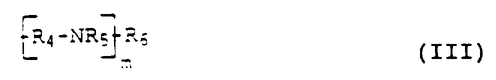
dans laquelle les restes R₄ sont mutuellement identiques ou différents et les restes R₅ sont mutuellement identiques ou différents et les restes R₄, R₅ et R₆ possèdent les significations qui leur ont été précédemment attribuées, et m représente un nombre entier dont la valeur varie de 1 à 7,
ou un radical polyoxyalkylène de la formule générale IV : 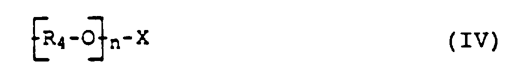
dans laquelle les restes R₄ peuvent être mutuellement identiques ou différents et posséder les significations qui leur ont été précédemment attribuées, X représente un radical alkyle en C₁ à C₆, ou un atome d'hydrogène et n représente un nombre entier dont la valeur varie de 1 à 30,
ou bien
dans laquelle R₂ et R₃ forment ensemble avec l'atome d'azote, auquel ils sont attachés, un radical morpholinyle, un radical pyridyle, un radical pipéridyle, un radical pyrrolyle, un radical pyrimidinyle, un radical pyrrolinyle, un radical pyrrolidinyle, un radical pyrazinyle, ou un radical pyridazinyle.

3. Composition de carburant ou de lubrifiant suivant la revendication 1, contenant au moins une poly-1-n-alcénamine de la formule générale I : 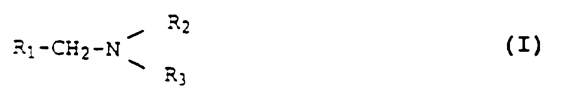 dans laquelle
R₁ représente un radical poly-1-n-alcéne dérivé d'un ou plusieurs 1-n-alcènes comportant de 3 à 6 atomes de carbone et de 0 à 50% en poids d'éthène, avec de 20 à 400 atomes de carbone, et
R₂ et R₃, qui peuvent être identiques ou différents, représentent chacun un atome d'hydrogène, un radical alkyle en C₁ à C₁₀, phényle, naphtyle, hydroxyalkyle en C₁ à C₁₀,
un radical aminoalkylène de la formule générale II : 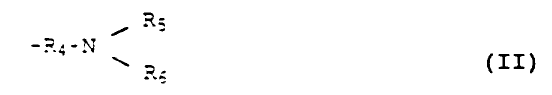
dans laquelle
R₄ représente un radical alkylène en C₂ à C₁₀, et
R₅ et R₆, qui sont identiques ou différents, représentent chacun un atome d'hydrogène, un radical alkyle en C₁ à C₁₀, phényle, naphtyle ou hydroxyalkyle en C₁ à C₁₀,
un radical polyaminoalkylène de la formule générale III : 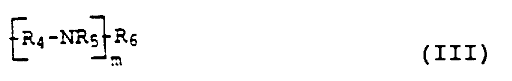
dans laquelle
les restes R₄ sont mutuellement identiques ou différents,
les restes R₅ sont mutuellement identiques ou différents,
les restes R₄ , R₅ et R₆, possèdent les significations qui leur ont été précédemment attribuées, et m représente un nombre entier dont la valeur varie de 1 à 7,
ou
un radical polyoxyalkylène de la formule générale IV : 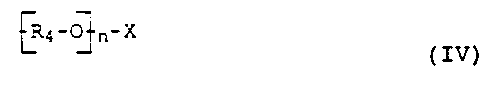
dans laquelle les restes R₄ sont mutuellement identiques ou différents et possèdent les significations qui leur ont été précédemment attribuées, X représente un radical alkyle en C₁ à C₆ ou un atome d'hydrogène et n représente un nombre entier dont la valeur varie de 1 à 30,
ou bien dans laquelle R₂ et R₃ forment ensemble avec l'atome d'azote, auquel ils sont attachés, un radical morpholinyle.

4. Composition de carburant ou de lubrifiant suivant la revendication 3, contenant au moins une poly-1-n-alcénamine de la formule générale I, dans laquelle le symbole R₁ représente un radical poly-1-n-alcène dérivé d'un ou plusieurs 1-n-alcènes comportant 3 à 4 atomes de carbone et 0 à 40% en poids d'éthène, et
R₂ et R₃ sont identiques ou différents et représentent chacun un atome d'hydrogène, un radical méthyle, éthyle, n-propyle, i-propyle, n-butyle, sec.-butyle, i-butyle, tert.-butyle, pentyle, hexyle, phényle, 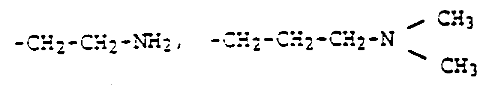 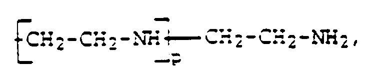
où p représente un nombre entier dont la valeur varie de 1 à 7, plus particulièrement, 1 à 3, 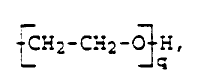
où q représente un nombre entier dont la valeur varie de 1 à 30, ou représentent ensemble avec l'atome d'azote, auquel ils sont attachés, un radical morpholinyle.

5. Composition de carburant ou de lubrifiant suivant la revendication 1, contenant au moins une poly-1-n-alcénamine de la formule générale I, dans laquelle le symbole R₁ représente un radical poly-1-n-alcène dérivé du propène, ou d'un mélange d'éthène et de 1-n-butène.

6. Composition de carburant ou de lubrifiant suivant la revendication 1, caractérisée en ce que l'on peut obtenir la poly-1-n-alcénamine par
a) la polymérisation de 1-n-alcènes en présence d'un catalyseur du type métallocènes de la formule générale V :
CpₘMXₙYᵣ (V)
dans laquelle Cp représente un noyau cyclopentadiène éventuellement substitué, M représente un métal de transition du groupe 4 b, X représente un atome d'hydrogène ou un radical alkyle en C₁ à C₆ et Y représente un atome d'halogène et dans laquelle m=1-3, n=0-3, r=0-3 et m+n+r correspondent à la valence de M,
b) l'hydroformylation subséquente du poly-1-n-alcène, et
c) l'amination hydrogénante du produit de la réaction issu de l'étape réactionnelle b).

7. Composition de carburant suivant la revendication 1, caractérisée en ce que qu'elle contient la poly-1-n-alcénamine en une proportion de 10 à 5000 ppm, plus particulièrement de 100 à 800 ppm, par rapport au poids total de la composition.

8. Composition de lubrifiant suivant la revendication 1, caractérisée en ce qu'elle contient la poly-1-n-alcénamine en une proportion de 0,5 à 5% en poids, plus particulièrement de 1 à 3% en poids, par rapport au poids total de la composition.

9. Poly-1-n-alcénamines de la formule générale I : 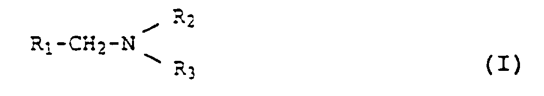 dans laquelle R₁ représente un radical poly-1-n-alcène dérivé d'un ou plusieurs 1-n-alcènes comportant de 3 à 6 atomes de carbone et de 0 à 50% en poids d'éthène, et
R₂ et R₃, qui sont identiques ou différents, représentent chacun
un atome d'hydrogène, un radical alkyle en C₁ à C₁₀, phényle, naphtyle, hydroxyalkyle en C₁ à C₁₀,
un radical aminoalkylène de la formule générale II : 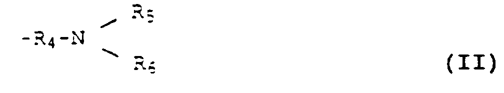
dans laquelle
R₄ représente un radical alkylène en C₁ à C₁₀, et R₅ et R₆, qui sont identiques ou différents, représentent chacun un atome d'hydrogène, un radical alkyle en C₁ à C₁₀, phényle, naphtyle ou hydroxyalkyle en C₁ à C₁₀,
un radical polyaminoalkylène de la formule générale III : 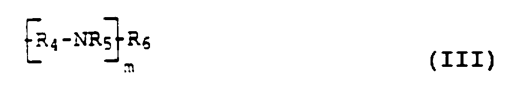
dans laquelle les restes R₄ sont mutuellement identiques ou différents, les restes R₅ sont mutuellement identiques ou différents, les restes R₄ , R₅ et R₆ possèdent les significations qui leur ont été précédemment attribuées, et m représente un nombre entier dont la valeur varie de 1 à 7, ou
un radical polyoxyalkylène de la formule générale IV : 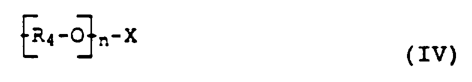 dans laquelle les restes R₄ sont mutuellement identiques ou différents et possèdent les significations qui leur ont été précédemment attribuées, X représente un radical alkyle en C₁ à C₆ ou un atome d'hydrogène et n représente un nombre entier dont la valeur varie de 1 à 30,
ou dans laquelle R₂ et R₃ forment ensemble avec l'atome d'azote, auquel ils sont attachés, un noyau dans lequel encore d'autres hétéroatomes peuvent être présents.

10. Utilisation des poly-1-n-alcénamines de la formule générale I suivant la revendication 9, à titre d'additifs pour des compositions de carburant ou de lubrifiant, plus particulièrement pour des moteurs à combustion.
